# EUROPEAN PATENT SPECIFICATION

(11) **EP 1 769 770 B1**
(45) Date of publication and mention of the grant of the patent: **17.03.2010**
(21) Application number: 06120906.0
(22) Date of filing: 17.11.2003
(51) Int. Cl.: A61B 19/00, A61B 10/02

(54) **Apparatus for delivering a tissue localizing and marking device**
Gerät zur Einführung einer Vorrichtung zur Markierung und Lokalisierung eines Gewebes
Appareil pour acheminer un dispositif de repérage et de marquage de tissu

(30) Priority: 18.11.2002 US 427048
(43) Date of publication of application: 04.04.2007
(62) Divisional of application: 03783502.2
(73) Proprietor: Bard Peripheral Vascular, Inc., Tempe, AZ 85280-1740 (US); Bard Shannon Limited, 3431 HR Nieuwegein (NL)
(72) Inventor: CHESBROUGH, Richard M., Bloomfield Hills, MI 48301 (US); FIELD, Steven E., Grand Rapids, MI 49546 (US); GOOSEN, Ryan L., Coopersville, MI 49404 (US)
(74) Representative: Tomlinson, Edward James

(56) References cited:
- EP-A- 1 163 888
- EP-A2- 0 769 281
- WO-A-01/00101
- WO-A-01/08578
- US-B1- 6 234 177

## Description

### BACKGROUND OF THE INVENTION

### Field of the Invention

The invention relates generally to a combined percutaneous imaging marker and localizing wire for imaging and surgically identifying an area of interest within a tissue mass. More particularly, the invention relates to a combined percutaneous imaging marker and localizing wire having a connection enabling separation of the localizing wire from the imaging marker after placement of the imaging marker in the tissue mass.

### Description of the Related Art

Tissue biopsies utilizing a biopsy cannula are a well-known procedure for diagnosing the presence of a malignancy in a tissue mass comprising a tissue anomaly, such as a lesion. If the results of the biopsy indicate the presence of a malignancy, a follow-up surgical procedure involving excision of the lesion is frequently performed. In certain tissue masses, such as breast tissue, it may be difficult to locate the lesion through palpation or visual observation. Contemporary imaging techniques can image a lesion slightly larger than the size of a subsequent biopsy specimen. Thus, after the biopsy specimen is taken, the remaining lesion can be too small to be imaged, making it difficult to find the lesion for surgical excision.

This is particularly true of suspected breast tissue malignancies, where the accepted medical approach is to evaluate and, if necessary, treat the suspected malignancy at the earliest possible time. To ensure that the lesion can be located after the biopsy, a marker is frequently inserted into the tissue mass through the biopsy cannula to locate the lesion for further evaluation using imaging techniques, such as radiography, ultrasound, or MRI. The sole purpose of the marker is to provide an imageable reference for locating the lesion.

If subsequent evaluation of the biopsy sample reveals the absence of a malignancy, the marker is typically used for follow-up evaluation at a subsequent time, which may be several months, to confirm either the continued absence of a malignancy or the development of a further change in the tissue mass indicating the need for further evaluation.

If subsequent evaluation of the biopsy sample reveals the presence of a malignancy, surgery is frequently performed. While the marker can locate the lesion through imaging techniques, it is inadequate as a guide for the surgeon to quickly locate the lesion with minimal trauma to the surrounding tissue. Consequently, a localizing wire is typically inserted with the marker being used to guide the insertion of the localizing wire. The localizing wire has a metal shaft that terminates in a folded portion forming an anchor for anchoring the localizing wire in the tissue mass. The shaft extends from the anchoring structure to the exterior of the body to be followed by the surgeon in locating the lesion. The localizing wire is typically removed during the surgery.

The decision to perform surgery is typically made after the biopsy has been taken and the marker has been placed. Thus, it is necessary to place the localizing wire in a separate procedure, again involving the insertion of a cannula into the tissue mass. This can be an extremely painful procedure for the patient. The procedure is also time-consuming and costly. Additionally, while the marker is used to relocate the lesion for placement of the localizing wire, it is still possible to mislocate the localizing wire, particularly if the marker has moved. In such a case, the lesion may not be sufficiently excised, or excess, healthy tissue may be unnecessarily excised.

It would be convenient for the patient if the separate insertion procedure for the localizing wire could be eliminated upon a determination that the biopsy specimen was malignant. It would be an improvement, both in cost and patient health, if the surgical excision of the lesion could be accomplished without the intervening insertion of the localizing wire.

International Application Publication No. WO 01/00101 A1 ("Burbank") discloses a biopsy site marker applicator apparatus that includes an elongated cylindrical body having an interior cavity and a piston that fits and slides back and forth in the elongated cylindrical body. The cylindrical body can be an elongated flexible tube. The applicator can be loaded with a desired number of biopsy markers comprising pellets having gelatin bodies. The pellets are expelled one-by-one from the cylinder as the piston is pushed into the cylinder or tube.

WO 01/08578 describes a variety of biopsy makers and an apparatus for inserting a marker into a patient's tissue comprising a cannula with the marker contained in the cannula. The marker is pushed out of an end of the cannula using a pusher device.

EP 1 163 888 A1 describes a variety of biopsy markers and an apparatus for placing the markers in position in a patient's tissue. The apparatus comprises a cannula with the marker being ejected from the cannula tip by the action of a stylet within the cannula.

US 6,234,177 B1 describes an apparatus for deploying an expandable biopsy marker in which the marker is ejected from an aperture in the side of a deployment catheter by the action of a plunger in a catheter lumen.

### SUMMARY OF THE INVENTION

The present invention provides a tissue localizing and marking apparatus comprising an introducer, an imaging element and a guide element according to claim 1. Preferred aspects of the invention are provided according to the dependent claims.

The imaging element can be imaged by any imaging techniques, an illustrative listing of which includes: x-ray, ultrasound, magnetic resonance imaging (MRI), computer assisted tomography (CAT), mammography, fluoroscopy, Doppler, other roentgenological imaging methods, visualization, and detection of emission. The imaging element and guide element can comprise a portion that is one ofbioabsorbable or non-bioabsorbable. The imaging and guide elements can be entirely bioabsorbable or non-bioabsorbable.
Preferably, the imaging element comprises a first portion that is non-bioabsorbable and a second portion that is bioabsorbable. The first portion can be made of a suitable metal, such as titanium, and the second portion can be made of a material such as collagen.
The imaging element can further comprise at least one extension, with a bioabsorbable element mounted to the extension. The bioabsorbable element can encapsulate at least a portion of the extension.
The imaging element can define a guide element opening through which the guide element passes. Preferably, a loop formed in the imaging element defines the guide element opening. The guide element is preferably a filament, which can be made from a bioabsorbable material or a suture material. The filament can alternatively be a metal wire.
A holder can be mounted to a portion of the guide element exterior of the tissue mass to hold the position of the guide element relative to the tissue mass.
A releasable connection can connect the guide element to the imaging element to define the separable portion. The releasable connection can comprise a threaded coupling, a weldment, a severable portion of the guide element, or a discontinuity in the guide element.
A gripping element can be attached to the guide element for separating the guide element from the imaging element.

### BRIEF DESCRIPTION OF THE DRAWINGS

In the drawings:
Figure 1 is an elevational view of an introducer for inserting a medical device in the form of a combination localizing and marking apparatus according to the invention.
Figure 2 is an enlarged sectional view of a portion of the introducer of Figure 1 showing the combination localizing and marking apparatus therein contained within a recess in the introducer.
Figure 3 is a perspective view of the combination localizing and marking apparatus shown in Figure 2 comprising an embodiment of an imaging element and a guide element, with the guide element being removable from the imaging element through a releasable connection.
Figure 4A is an enlarged view of the imaging element shown in Figure 3 illustrating a first embodiment of the releasable connection.
Figure 4B is an enlarged view of the imaging element shown in Figure 3 illustrating a second embodiment of the releasable connection.
Figure 4C is an enlarged view of the imaging element shown in Figure 3 illustrating a third embodiment of the releasable connection and an alternate embodiment of the guide element comprising a filament loop.
Figure 5A is an enlarged view of a second embodiment of the imaging element shown comprising a non-bioabsorbable portion and a bioabsorbable portion.
Figure 5B is an enlarged view of a third embodiment of the imaging element comprising a non-bioabsorbable portion and a bioabsorbable portion, and an alternate embodiment of the guide element.
Figure 5C is an enlarged view of a fourth embodiment of the imaging element comprising a non-bioabsorbable portion and a bioabsorbable portion.
Figure 5D is an enlarged view of the embodiment of the imaging element shown in Figure 3 comprising a non-bioabsorbable portion and a bioabsorbable portion.
Figure 5E is an enlarged view of a fifth embodiment of the imaging element comprising a non-bioabsorbable portion and a bioabsorbable portion.
Figure 6A is a perspective view illustrating the insertion of the introducer into breast tissue to mark and localize a tissue anomaly in the form of a lesion with the combination localizing and marking apparatus.
Figure 6B is a perspective view illustrating the initiation of the expulsion of the combination localizing and marking apparatus at the lesion once the introducer is properly located relative to the lesion.
Figure 6C is a perspective view illustrating the complete expulsion of the the combination localizing and marking apparatus at its placement at the lesion in the breast tissue mass.
Figure 7 is an enlarged view of the combination localizing and marking apparatus after placement in the tissue mass and withdraw of the introducer and showing the imaging element at the lesion and the guide element extending exteriorly of the body.
Figure 8 is an enlarged view of the combination localizing and marking apparatus of Figure 3 showing the imaging element at the lesion and the guide element being removed from the tissue mass after separation from the imaging element at the releasable connection.
Figure 9 is an enlarged view of a sixth embodiment of the imaging element comprising a repositionable imaging element comprising a non-bioabsorbable portion and a bioabsorbable portion.

### DESCRIPTION OF EMBODIMENTS OF THE INVENTION

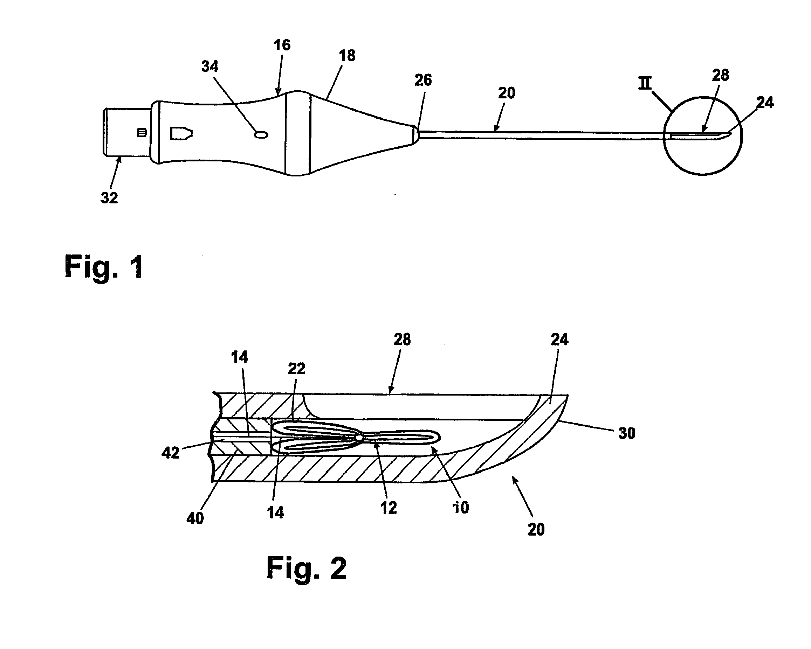

Referring now to the drawings, and specifically to Figures 1-3, an introducer 16 housing a medical device 10 according to the invention. The medical device 10 is shown comprising an imaging element 12 and a guide element 14. The medical device 10 is placed in a tissue mass with the introducer 16, which may comprise any well-known introducing device sufficient for the purposes described herein.

For illustration purposes, the introducer 16 is shown comprising a handle 18 and a cannula 20. As shown in Figure 1, the cannula 20 has a distal end 24, and a proximal end 26 attached to the handle 18. The cannula 20 comprises a lumen 22 through which the medical device 10 is conveyed for placement in the tissue mass. The introducer 16 also comprises a generally well-known plunger 32 that is slidable relative to the handle 18.

The cannula 20 is shown in Figures 1 and 2 terminating at the distal end 24 in a closed insertion tip 30 which is adapted in a well-known manner for ease of insertion of the cannula 20 into the tissue mass. Adjacent the insertion tip 30 is an expulsion opening 28 in the wall of the cannula 20 in fluid communication with the lumen 22 adapted for expulsion of the medical device 10 from the lumen 22. A piston 40, referred to herein as a stylet, is in slidable register with the lumen 22. A proximal end of the stylet 40 is mounted to the plunger 32, such that the slidable movement of the plunger 32 effects a similar movement of the stylet 40. A distal end of the stylet 40 is spaced from the insertion tip 30 to define a recess within the cannula 20 that receives the medical device 10 as best seen in Figure 2. The sliding of the plunger32 relative to the handle 18 slides the plunger 32 into the recess and forces the medical device 10 out the expulsion opening 28.

While the cannula 20 is shown with a side or lateral expulsion opening 28, the cannula 20 could just as easily have an end or axial opening formed by extending the lumen through the insertion tip 30. With this configuration, the medical device 10 is axially expelled instead of laterally expelled from the cannula 20.

As shown in Figure 2, a guide element bore 42 extends coaxially through the stylet 40 for receipt of the guide element 14 as hereinafter described. The handle 18 is provided with a guide element aperture 34 in fluid communication with the guide element bore 42 for receipt of the guide element 14. Alternatively, the stylet 40 can be provided with a radial slot (not shown) extending longitudinally the length of the stylet 40 and fluidly communicating with the guide element aperture 34 for receipt of the guide element 14, rather than the guide element bore 42.

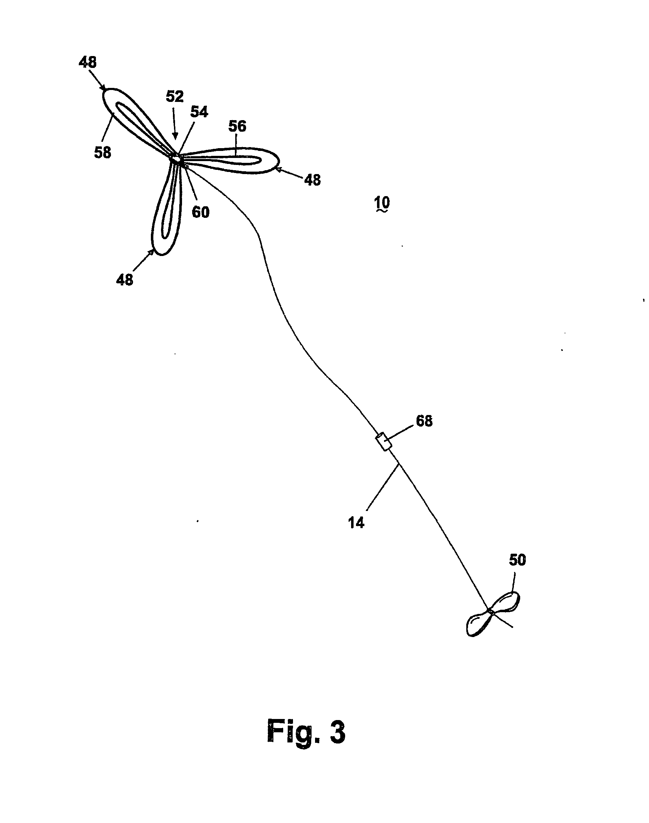

Referring now specifically to Figure 3, a first embodiment 52 of the imaging element is shown for illustrative purposes as comprising three radially-aligned, elongated extensions 48, each comprising a non-bioabsorbable portion 56 and a bioabsorbable portion 58 regularly spaced about a center hub 54. The elongated extensions 48 preferably perform dual functions of being imageable and anchoring the imaging element 52 in the tissue mass. The bioabsorbable portion 58 is used to enhance the imageability of the imaging element 52 in an imaging method different from the extensions 48.

For example, the non-bioabsorbable portion 56 can comprise a metal, such as titanium, tungsten or a ferromagnetic material, and is preferably most easily imageable using x-ray, while the bioabsorbable portion 58 can be collagen or gelatin, which is most easily imageable using ultrasound. While these materials are preferred, the imaging element 52 can be made from any suitable imaging material and imaged using any one of the following common imaging techniques: x-ray, ultrasound, magnetic resonance imaging (MRI), computer assisted tomography (CAT), mammography, fluoroscopy, Doppler, other roentgenological imaging methods, visualization, and detection of emission. Unless otherwise indicated, further references to one or more specific imaging techniques is intended for illustrative purposes and not as a limitation.

Although Figure 3 illustrates an imaging element 52 comprising a non-bioabsorbable portion 56 and a bioabsorbable portion 58, the imaging element 52 can be fabricated entirely of a non-bioabsorbable material, or entirely of a bioabsorbable material. If the imaging element 52 is fabricated entirely of a non-bioabsorbable material, imaging may be restricted to a specific techniques such as radiography. Alternatively, if the imaging element 52 is fabricated entirely of a bioabsorbable material, imaging may be restricted to ultrasound. The selection of a non-bioabsorbable material and/or a bioabsorbable material for the imaging element 52 is expected to take into account the future disposition of the imaging element 52 in the tissue mass, as well as the preferred imaging technique. The bioabsorbable portion 56 is illustrated in the Figures as comprising a translucent material, which is the preferred embodiment. However, the bioabsorbable portion 56 can alternatively comprise an opaque material.

The guide element 14 is shown comprising a thin, filament body, such as a thin, titanium wire, a suture, or the like. It is anticipated that the guide element 14 will comprise a structure that functions to guide a surgeon to the biopsy site, in the same manner as a traditional localizing wire. If a suture material is used, it can comprise a bioabsorbable suture. The guide element 14 is attached to the imaging element 52 through a releasable connection 60 which is adapted for selective separation of the guide element 14 from the imaging element 52.

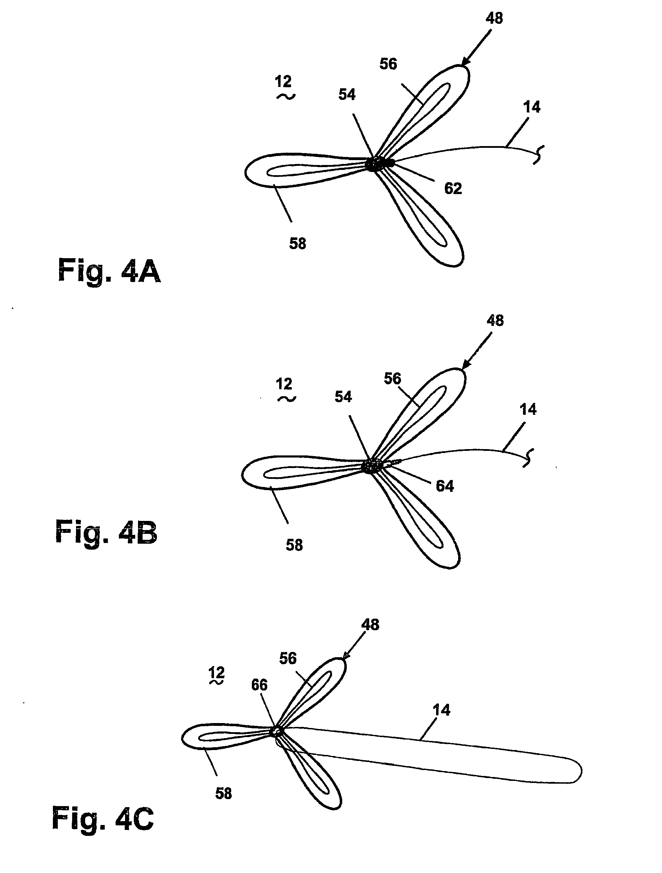

The releasable connection 60 can take one of several different structures. For example, as illustrated in Figure 4A, the releasable connection 60 can comprise a spot weld 62 on the guide element 14 at or near the hub 54. The spot weld 62 results in a localized weakness in the guide element 14 enabling the guide element 14 to be separated at that point. As illustrated in Figure 4B, the releasable connection 60 can also comprise a threaded connection 64, in which the end of the guide element 14 is provided with male threads and the hub 54 is provided with cooperating female threads. The guide element 14 is threaded to the imaging element 52 for insertion into the tissue mass. Twisting of the guide element 14 after insertion will unthread the guide element 14 from the imaging element 52 for removal of the guide element 14 from the tissue mass.

As illustrated in Figure 4C, the hub 54 can comprise a ring 66, or alternatively can be provided with a loop portion extending therefrom, through which the guide element 14 can be threaded, similar to the threading of a needle. The guide element 14 can comprise a filament loop passing through the ring 66 and extending exteriorly of the tissue mass. The guide element 14 can be separated from the imaging element 12 by cutting the filament loop to form two strands and then pulling on one of the strands.

Other suitable separation configurations can be employed, such as a notch, an indentation, or a narrowing of the guide element 14 at the desired point of separation, providing a localized weakness or discontinuity in the guide element 14. Twisting of the guide element 14 will separate the guide element 14 at the localized weakness, similar to the spot weld. Alternatively, the guide element 14 can be separated by first depressing the tissue mass surrounding the guide element 14 in order to reveal a sufficient length of the guide element 14 otherwise engulfed within the tissue mass. The guide element 14 can then be separated at a point adjacent the depressed tissue mass by severing the guide element 14. When the tissue mass rebounds, no portion of the guide element 14 will extend beyond the tissue mass. With such a configuration, it is preferred that the guide element be bioabsorbable. It will be understood that the separation techniques described herein are intended to be illustrative only, and that other separation techniques are feasible and are within the scope of the herein-described inventive concept.

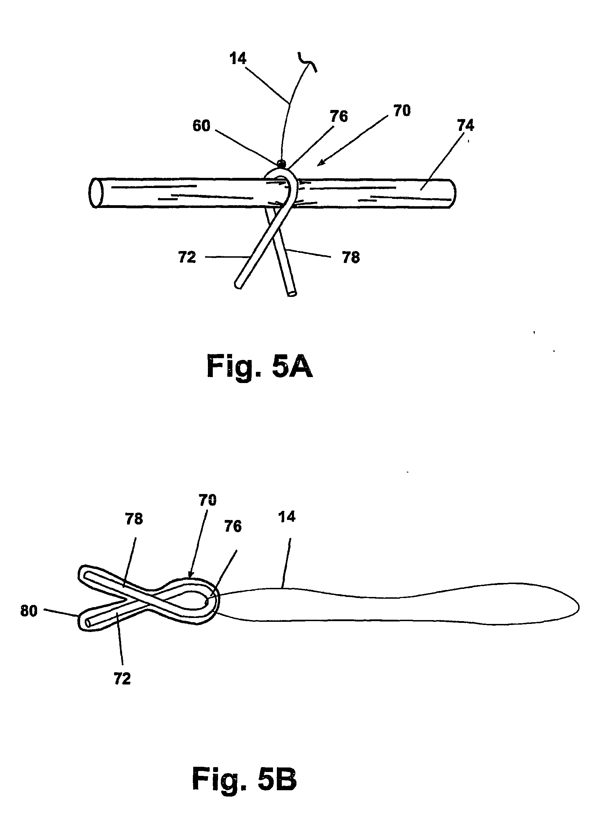

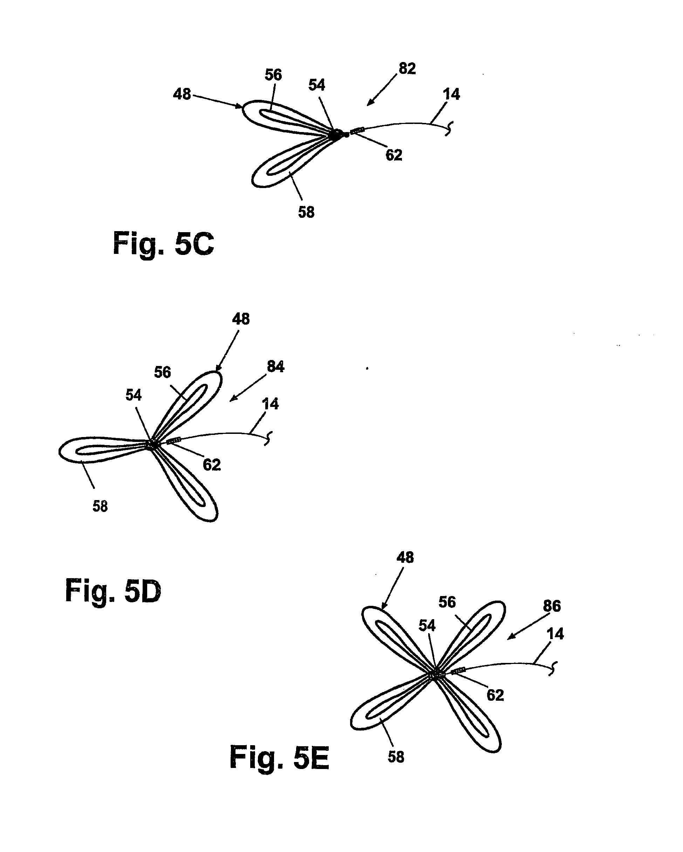

Figures 5A-E illustrate several different embodiments of the imaging element 12. Figure 5A illustrates an imaging element 70 having fine general shape of the Greek letter alpha, and comprising a loop 76 of non-bioabsorbable, wire-like material 72 terminating in a pair of legs 78. The guide element 14 is connected to the imaging element 70 through one of the aforementioned releasable connections 60. The releasable connection 60 can be located at the loop 76, one of the legs 78, or other suitable location on the non-bioabsorbable portion 72. Another non-bioabsorbable portion 74 comprises a flexible, elongated, cylindrical rod adapted for insertion through the loop 76. The second non-bioabsorbable portion 74 can be folded and/or compressed for insertion into the cannula 20, to be expanded when the medical device 10 is ejected into the tissue mass. The non-bioabsorbable portion 74 is preferably a foam material having air pockets that are very imageable using ultrasound techniques, as previously discussed, whereas the metal alpha-shaped portion is very imageable using x-ray. Alternatively, the rod-shaped portion 74 can comprise a bioabsorbable material that can be imaged using ultrasound techniques.

Figure 5B illustrates an alpha-shaped imaging element 70 comprising a loop 76 of the non-bioabsorbable, wire-like material 72 terminating in a pair of legs 78. Again, the guide element 14 is connected to the imaging element 70 through one of the aforementioned releasable connections 60. A bioabsorbable portion 80 comprises a bioabsorbable material, such as collagen or gelatin, applied in a generally uniform coating over the entire non-bioabsorbable portion 72. Alternatively, the bioabsorbable portion 80 can comprise a coating extending only over a portion of the imaging element 70, such as the legs 78. Figure 5B illustrates a releasable connection 60 comprising a filament loop extending through the loop 76 in a manner similar to the configuration illustrated in Figure 4C.

Figures 5C-E illustrate variations of the imaging element 12 illustrated in Figure 3, comprising a dual extension imaging element 82 (Figure 5C), a treble extension imaging element 84 (Figure 5D), and a four bladed imaging element 86 (Figure E). The guide element 14 can be connected to the imaging elements 82-86 through any of the releasable connections previously described herein.

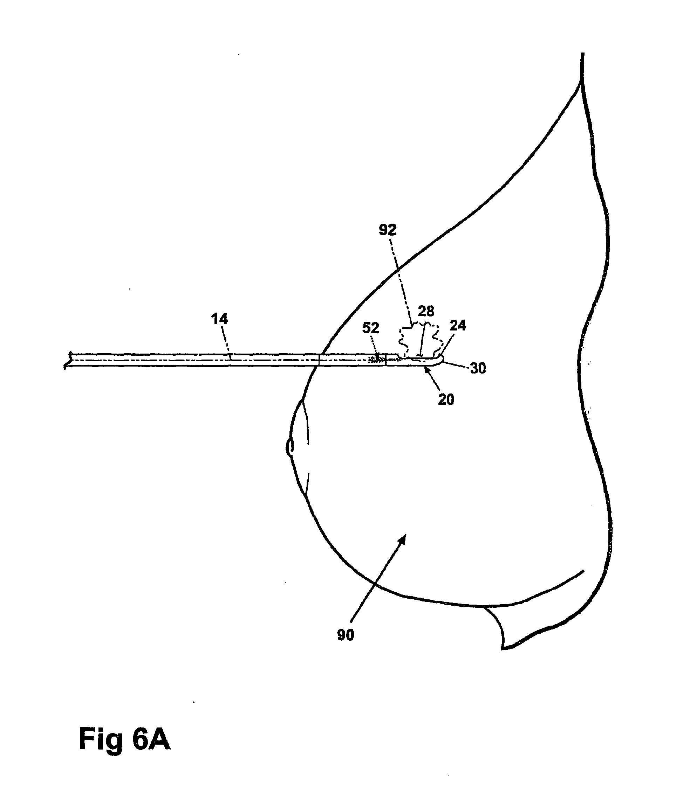

Figures 6A-C illustrate the placement of the medical device 10 at a biopsy site 92 in a tissue mass 90. Initially, as illustrated in Figures 1 and 2, the medical device 10 is inserted into the cannula 20 in preparation for ejection through the expulsion opening 28 with movement of the stylet 40 through the action of the plunger 32. Preferably, the introducer 16 is manufactured with the imaging element 12 loaded in the recess, such that the medical technician does not have to load the imaging element 12.

As illustrated, when loaded, the guide element 14 extends through the guide element bore 42 and out the guide element aperture 34. However, the guide element 14 could also have the end opposite the imaging element 12 freely contained within the guide element bore 42 or releasably attached to the stylet 40.

As illustrated in Figure 6A, the cannula 20 is inserted into the tissue mass 90 until the insertion tip 30 and the expulsion opening 28 are adjacent the biopsy site 92. It is anticipated that this procedure will immediately follow the recovery of a biopsy sample from the biopsy site 92, and that the cannula 20 will be inserted through a biopsy cannula (not shown) previously emplaced during the biopsy procedure. However, the cannula 20 can be inserted directly into the tissue mass 90 without the use of a biopsy cannula.

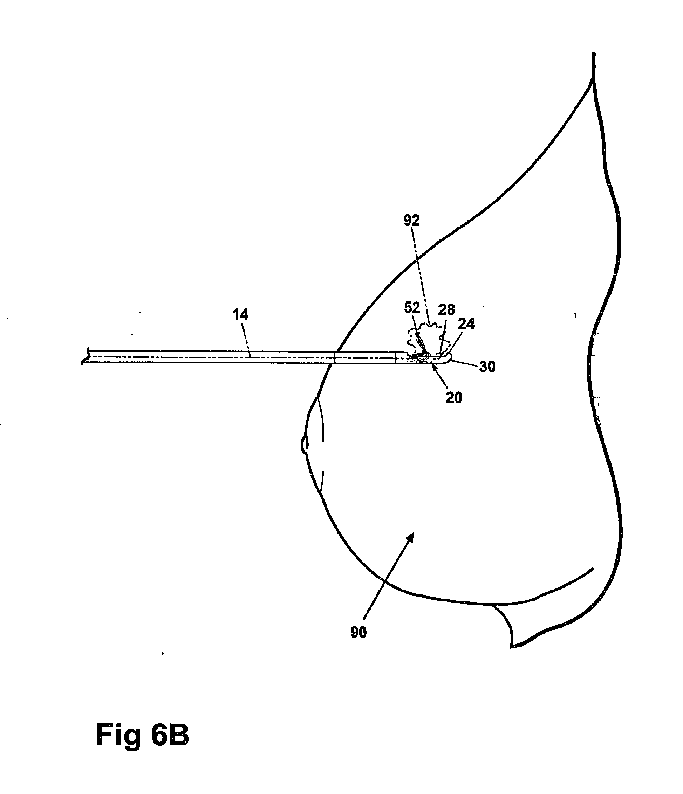

Once the cannula 20 is properly positioned, the imaging element 52 is then ejected through the expulsion opening 28 by sliding the plunger 32 which urges the stylet 40 toward the expulsion opening 28, thereby ejecting the imaging element 52 at the biopsy site 92, as illustrated in Figure 6B.

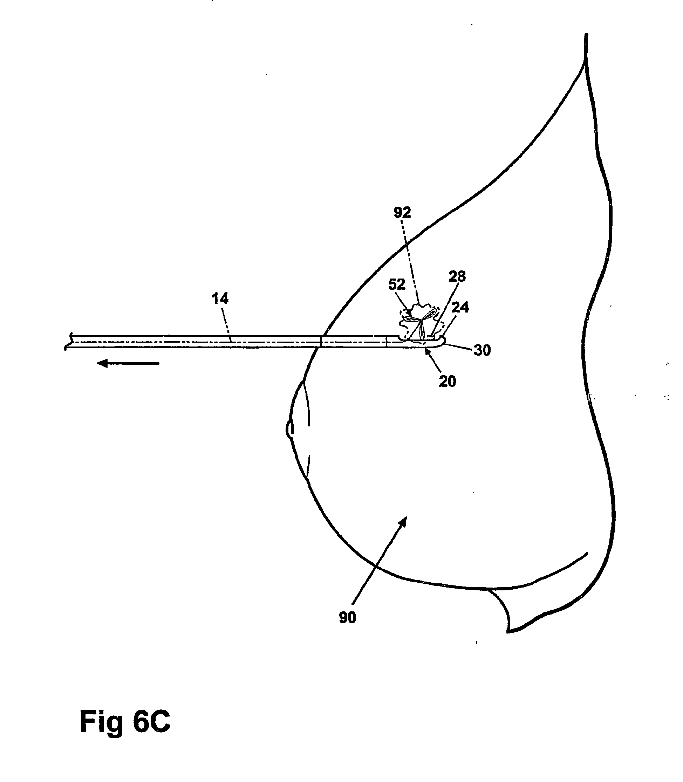

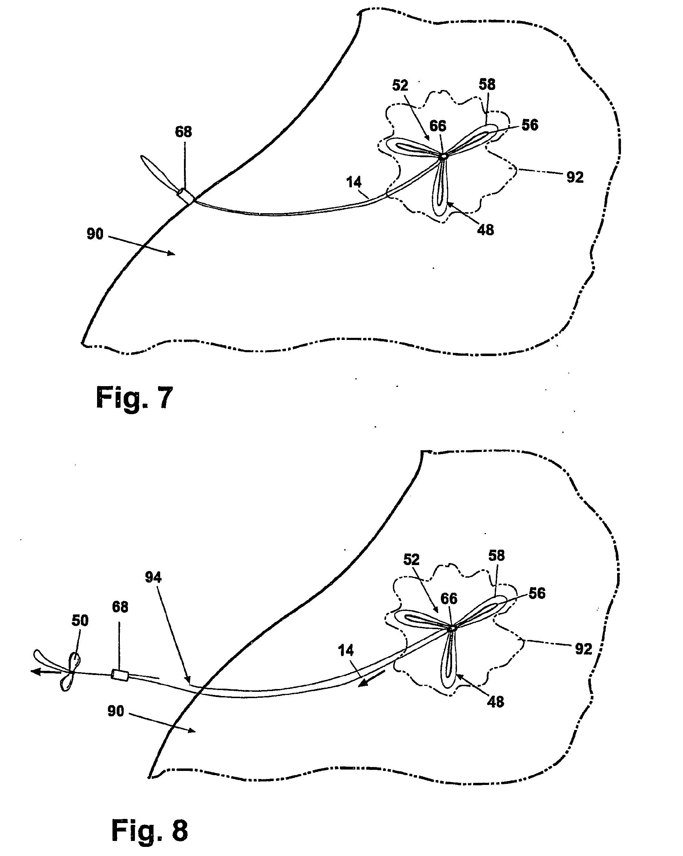

The cannula 20, and the biopsy cannula if present, is then removed from the tissue mass 90, as illustrated in Figure 6C, leaving the imaging element 52 in place and the guide element 14 extending through the cannula insertion line to the exterior of the tissue mass 90. The guide element 14 can be further secured in place by a holder 68, also referred to as a clip, which is positioned around the guide element 14 exterior of and in contact with the tissue mass, as shown in Figure 7.

The emplaced medical device 10 is illustrated in Figure 7. If further diagnosis indicates that surgical intervention is unnecessary, the guide element 14 can be separated from the imaging element 52 by attaching a guide element grip 50 to the guide element 14 to aid in removing the guide element 14. As an illustration, the guide element 14 is shown in Figure 8 as a loop, one strand of which is severed at a separation point 94. The guide element grip 50 can be attached to the external end of the loop to facilitate the pulling of the guide element 14 from the ring 66. The imaging element 52 will remain in place for future imaging, if necessary. The bioabsorbable portion 58 will be slowly adsorbed into the surrounding tissue mass 90, leaving the non-bioabsorbable portion 56 in place.

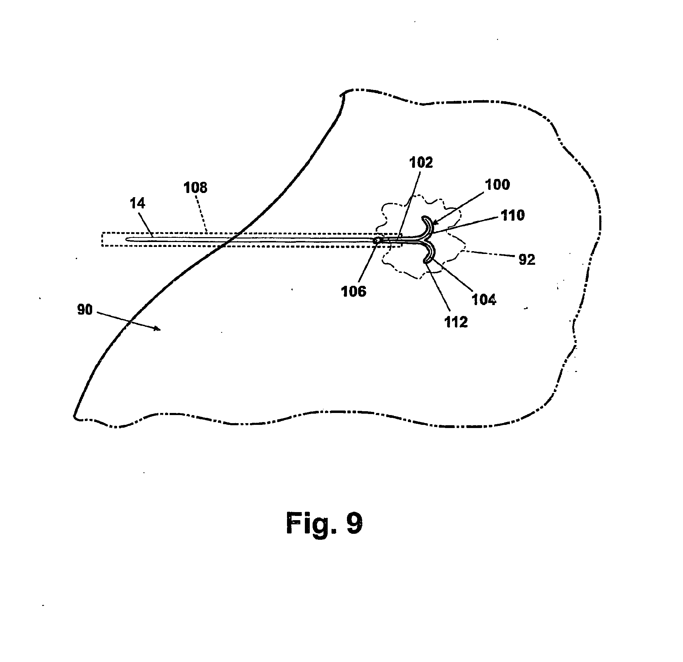

There are circumstances in which it may be necessary to reposition the medical device 10 after initial emplacement. For this purpose, a repositionable imaging element can be used. Illustrated in Figure 9 is a medical device comprising a repositionable imaging element 100 separably connected to the guide element 14, which is illustrated as a filament loop, through a releasable connection 106, illustrated as a ring-like body. The imaging element 100 is shown as a somewhat Y-shaped body comprising a shaft 102 terminating in a pair of opposed hooks 104. The imaging element 100 is illustrated as comprising a bioabsorbable portion 110 and a non-bioabsorbable portion 112 similar to the imaging elements previously described herein.

The non-bioabsorbable portion 112 preferably comprises a high-strength flexible metallic wire of suitable strength and flexibility for the purposes described herein, such as stainless steel, titanium, or a nickel-titanium alloy, such as Nitinol, which has shape memory characteristics. The bioabsorbable portion 110 comprises a material such as collagen or gelatin, as previously described herein. The non-bioabsorbable portion 112 has sufficient rigidity to securely anchored the imaging element 100 in the lesion 92. However, if it is necessary to reposition the medical device 10, the hooks 104 can deflect in order to disengage the imaging element 100 from the lesion 92 and enable the imaging element 100 to be repositioned.

The imaging element 100 is designed to be easily viewable under at least one of the common imaging techniques. In this manner, the imaging element is different from prior art hook devices that were used solely as anchors for a localizing wire. Advantageously, the imaging element 100 is several times larger than the previosly described imaging elements. The larger size of the imaging element 100 and the multiple hooks thus provide the imagable element 100 with a much greater holding force than the previously described imaging elements.

As shown in Figure 9, a well-known repositioning cannula 108 is typically utilized for the repositioning procedure. The cannula 108 is introduced into the tissue mass 90 with the guide element 14 extending therethrough until the cannula 108 encounters the imaging element 100. As the cannula 108 is further translated relative to the hooks 104, the hooks 104 engage the end of the cannula 108 and are elastically deflected. The imaging element 100 can be recovered into the lumen of the cannula 108 by continued insertion of the cannula 108 into the tissue mass 90, by pulling the guide element 14 to urge the imaging element 100 into the lumen, or a combination of both. With the imaging element 100 removed from the lesion 92 into the cannula 108, the cannula 108 can be repositioned for reinsertion of the imaging element 100 into the lesion. Should further medical evaluation indicate no need for surgical intervention, the guide element 14 can be removed from the imaging element 100 as previously described, which enables the complete removal of the medical device without separating the guide element from the imaging marker.

The repositionable imaging element 100 is typically larger than the imaging elements previously described herein. Thus, it may be desirable to recover the imaging element 100 from the lesion 92, rather than allowing the imaging element to remain in place as with the previously described embodiments. Thus, while the guide element 14 is attached to the imaging element 100 through the releasable connection 106 for separation of the guide element 14 from the imaging element 100 as previously described, the use of a repositionable imaging element 100 enables the medical device 10 to be recovered from the patient in its entirety. Furthermore, there may be unforeseen circumstances in which removal of the imaging element is very difficult or dangerous to the patient, or cannot be accomplished because of the failure of the releasable connection to properly separate. In anticipation of these circumstances, the repositionable imaging element 100 can be incorporated into the medical device 10 for both separation from the guide element 14, or complete removal.

The medical device 10 disclosed herein enables a physician to place, in effect, an imageable marker and a localizing wire at the same time during the core biopsy procedure. By placing the apparatus 10 at the site of the core biopsy, the site is marked with a secure device that can be easily visualized during subsequent imaging techniques. The separable guide element 14 is already in place shall a core biopsy pathology indicate surgical intervention. This eliminates the need for a separate wire localization procedure. If the core biopsy pathology indicates no need for surgical intervention, the physician can simply separate the guide element 14 from the imaging element 12, and remove the guide element 14 from the patient. The imaging element 12 remains in place to mark the site of the biopsy as with current imaging devices.

While the invention has been specifically described in connection with certain specific embodiments thereof, it is to be understood that this is by way of illustration and not of limitation. Reasonable variation and modification of embodiments described in the forgoing disclosure and drawings are possible without departing from the scope of the appended claims.

## Claims

1. A tissue localizing and marking apparatus comprising an introducer (16), an imaging element (12) and a guide element (14) both imaging element and guide element being contained in the introducer for the percutaneous placement thereof at a predetermined location in a tissue mass to facilitate subsequent determination of the predetermined location, the introducer comprising:
a cannula (20) having a distal end (24) and a proximal end (26), the cannula (20) comprising a peripheral wall forming a lumen (22), the distal end (24) terminating in a self-piercing tip (30);
a stylet (40) having a proximal end and a distal end, the stylet (40) being slidably received with the lumen (22) of the cannula (20) for movement between a ready position and an expelled position,
a plunger (32) mounted to the proximal end of the stylet (40) wherein a slidable movement of the plunger (32) effects a similar movement of the stylet (40); and
a handle (18) to be grasped by a user, the handle (18) being attached to the proximal end (26) of the cannula (20),
**characterized in that**
the imaging element (12) is coupled to the guide element (14),
the cannula comprises a lateral opening (28) in the peripheral wall, which is ' open to the lumen (22) and
in the ready position, the guide element (14) is received in a guide element aperture (34) of the handle (18)
and wherein in the ready position the distal end of the stylet (40) is spaced inwardly from the self-piercing tip (30) to form a marker recess that is in communication with the lateral opening (28) that is sized to receive the imaging element (12), and in the expelled position, the distal end of the stylet (40) is advanced a sufficient distance into the marker recess to expel the imaging element (12) contained therein through the lateral opening (28).

2. The apparatus according to claim 1, wherein the stylet (40) includes a guide element bore (42) extending coaxially through the stylet (40) for receipt of the guide element (14) and wherein the guide element aperture (34) is in fluid communication with the guide element bore (42).

3. The apparatus according to claim 1 or 2, configured such that, when the cannula (20) is removed from a tissue mass after the imaging element (12) is expelled, the guide element (14) extends exterior of the tissue mass.

4. The apparatus according to claim 1, wherein the stylet (40) includes a radial slot extending longitudinally along a length of the stylet (40) and wherein the radial slot fluidly communicates with the guide element aperture (34) for receipt of the guide element (14).

5. The apparatus according to claim 1, wherein the guide element (14) is attached to the imaging element (12) through a releasable connection (60).

6. The apparatus according to claim 1, wherein the handle (18), the cannula (20), the plunger (32) and the stylet (40) are operably coupled such that they form a self-contained marking apparatus (16) that can be easily and conveniently handled by a user to effect operation of the stylet (40) from the ready position to the expelled position.

7. The apparatus according to claim 1, wherein an end of the guide element (14) that is opposite the imaging element (12) is releasably attached to the stylet (40).

## Patentansprüche

1. Gerät zur Lokalisierung und Markierung von Gewebe mit einem Einführungsinstrument (16), einem Bildgebungsinstrument (12) und einem Führungselement (14), wobei sowohl das Bildgebungsinstrument als auch das Führungselement im Einführungsinstrument enthalten sind, um perkutan an einer vorab festgelegten Stelle in einer Gewebemasse platziert zu werden, um die anschließende Bestimmung der vorab festgelegten Stelle zu erleichtern, wobei das Einführungsinstrument Folgendes umfasst:
eine Kanüle (20) mit einem distalen Ende (24) und einem proximalen Ende (26), wobei die Kanüle (20) eine periphere Wand umfasst, die ein Lumen (22) formt, wobei das distale Ende (24) in einer selbststechenden Spitze (30) endet;
einen Dorn (40) mit einem proximalen Ende und einem distalen Ende, wobei der Dorn (40) gleitend für eine Bewegung zwischen einer Bereitschaftsstellung und einer ausgeschobenen Stellung im Lumen (22) der Kanüle (20) aufgenommen ist,
einen Kolben (32), der am proximalen Ende des Dorns (40) befestigt ist, worin eine Gleitbewegung des Kolbens (32) eine ähnliche Bewegung des Dorns (40) bewirkt; und
einen Handgriff (18), der von einem Anwender ergriffen wird, wobei der Handgriff (18) am proximalen Ende (26) der Kanüle (20) befestigt ist,
**dadurch gekennzeichnet, dass** das Bildgebungselement (12) mit dem Führungselement (14) verbunden ist, wobei die Kanüle eine seitliche Öffnung (28) in der peripheren Wand umfasst, die zum Lumen (22) hin offen ist, und das Führungselement (14) in der Bereitschaftsstellung in einer Führungselement-Öffnung (34) des Handgriffs (18) aufgenommen ist, und worin das distale Ende des Dorns (40) in der Bereitschaftsstellung nach innen von der selbststechenden Spitze (30) beabstandet ist, um eine Markierungsvertiefung zu formen, die mit der seitlichen Öffnung (28), die eine solche Größe aufweist, dass sie das Bildgebungselement (12) aufnehmen kann, in Verbindung steht, und worin das distale Ende des Dorns (40) in der ausgeschobenen Stellung ausreichend weit in die Markierungsvertiefung vorgeschoben wird, um das Bildgebungselement (12), das darin enthalten ist, durch die seitliche Öffnung (28) auszuschieben.

2. Gerät nach Anspruch 1, worin der Dorn (40) eine Führungselement-Bohrung (42) aufweist, die sich koaxial durch den Dorn (40) erstreckt, um das Führungselement (14) aufzunehmen, und worin die Führungselement-Öffnung (34) mit der Führungselement-Bohrung (42) in Flüssigkeitsverbindung steht.

3. Gerät nach Anspruch 1 oder 2, das so konfiguriert ist, dass das Führungselement (14) sich außerhalb der Gewebemasse erstreckt, wenn die Kanüle (20) nach dem Ausschieben des Bildgebungselements (12) aus einer Gewebemasse entfernt wird.

4. Gerät nach Anspruch 1, worin der Dorn (40) einen radialen Schlitz aufweist, der sich längs über eine Strecke des Dorns (40) erstreckt, und worin der radiale Schlitz mit der Führungselement-Öffnung (34) in Flüssigkeitsverbindung steht, um das Führungselement (14) aufzunehmen.

5. Gerät nach Anspruch 1, worin das Führungselement (14) durch eine lösbare Verbindung (60) mit dem Bildgebungselement (12) verbunden ist.

6. Gerät nach Anspruch 1, worin der Handgriff (18), die Kanüle (20), der Kolben (32) und der Dorn (40) so in Wirkverbindung stehen, dass sie ein abgeschlossenes Markierungsgerät (16) bilden, das von einem Anwender leicht und bequem gehandhabt werden kann, um die Bedienung des Dorns (40) aus der Bereitschaftsstellung in die ausgeschobene Stellung zu bewirken.

7. Gerät nach Anspruch 1, worin ein Ende des Führungselements (14), das dem Bildgebungselement (12) gegenüberliegt, lösbar am Dorn (40) befestigt ist.

## Revendications

1. Appareil pour acheminer un dispositif de repérage et de marquage de tissu comportant un introducteur (16), un élément (12) d'imagerie et un élément (14) guide, l'élément d'imagerie et l'élément guide étant tous deux contenus dans l'introducteur pour être positionnés de manière percutanée à un emplacement prédéterminé dans une masse tissulaire afin de déterminer plus facilement par la suite l'emplacement prédéterminé, introducteur comportant :
une canule (20) munie d'une extrémité distale (24) et d'une extrémité proximale (26), la canule (20) comportant une paroi périphérique formant une lumière (22), l'extrémité distale (24) se terminant en un bout autoperceur (30) ;
un stylet (40) muni d'une extrémité proximale et d'une extrémité distale, le stylet (40) étant reçu de manière coulissante dans la lumière (22) de la canule (20) pour se déplacer entre une position prête et une position expulsée,
un plongeur (32) monté sur l'extrémité proximale du stylet (40) dans lequel un déplacement coulissant du plongeur (32) entraîne un déplacement similaire du stylet (40) ; et
une poignée (18) destinée à être saisie par un utilisateur, la poignée (18) étant attachée à l'extrémité proximale (26) de la canule (20),
**caractérisé en ce que**
l'élément (12) d'imagerie est accouplé à l'élément (14) guide,
la canule comporte une ouverture latérale (28) dans la paroi périphérique, qui est ouverte à la lumière (22) et
dans la position prête, l'élément (14) guide est reçu dans un jour (34) de l'élément guide de la poignée (18)
et dans lequel, dans la position prête, l'extrémité distale du stylet (40) est espacée vers l'intérieur depuis le bout autoperceur (30) pour former un renfoncement de marqueur qui est en communication avec l'ouverture latérale (28) qui est dimensionnée pour recevoir l'élément (12) d'imagerie, et dans la position expulsée, l'extrémité distale du stylet (40) est avancée d'une distance suffisante dans le renfoncement de marqueur pour expulser l'élément (12) d'imagerie qui y est contenu à travers l'ouverture latérale (28).

2. Appareil selon la revendication 1, dans lequel le stylet (40) comprend un alésage (42) d'élément guide s'étendant coaxialement dans le stylet (40) pour recevoir l'élément (14) guide et dans lequel le jour (34) de l'élément guide est en communication fluidique avec l'alésage (42) de l'élément guide.

3. Appareil selon la revendication 1 ou 2, configuré de telle sorte que, lorsque la canule (20) est enlevée d'une masse tissulaire après l'expulsion de l'élément (12) d'imagerie, l'élément (14) guide s'étend à l'extérieur de la masse tissulaire.

4. Appareil selon la revendication 1, dans lequel le stylet (40) comprend une fente radiale s'étendant longitudinalement sur une longueur de stylet (40) et dans lequel la fente radiale communique de manière fluidique avec le jour (34) de l'élément guide pour recevoir l'élément (14) guide.

5. Appareil selon la revendication 1, dans lequel l'élément (14) guide est attaché à l'élément (12) d'imagerie par un raccord (60) libérable.

6. Appareil selon la revendication 1, dans lequel la poignée (18), la canule (20), le plongeur (32) et le stylet (40) sont accouplés de manière fonctionnelle de telle sorte qu'ils forment un appareil (16) de marquage autonome qui peut être aisément et commodément manipulé par un utilisateur pour faire fonctionner le stylet (40) de la position prête à la position expulsée.

7. Appareil selon la revendication 1, dans lequel une extrémité de l'élément (14) guide qui est opposée à l'élément (12) d'imagerie est attachée de manière libérable au stylet (40).
